# EUROPEAN PATENT APPLICATION

(11) **EP 1 627 877 A1**
(43) Date of publication of application: **22.02.2006**
(21) Application number: 04729194.3
(22) Date of filing: 23.04.2004
(51) Int. Cl.: C07D 471/06, C07D 231/56

(54) **PROCESSES FOR PRODUCING PYRAZOLOACRIDONE DERIVATIVE AND INTERMEDIATE THEREFOR**

(30) Priority: 24.04.2003 JP 2003119943
(71) Applicant: KYOWA HAKKO KOGYO CO., LTD., Chiyoda-ku, Tokyo 100-8185 (JP)
(72) Inventor: TSUBAKIHARA, Nobuaki Kyowa Hakko Kogyo Co., Ltd., Osaka 590-8554 (JP); KATSUHIRA, Takeshi Kyowa Hakko Kogyo Co., Ltd., Osaka 590-8554 (JP); KINUGAWA, Masahiko Kyowa Hakko Kogyo Co., Ltd., Osaka 590-8554 (JP); KATO, Nobuyuki Kyowa Hakko Kogyo Co., Ltd., Osaka 590-8554 (JP)
(74) Representative: Casalonga, Axel
(86) International application number: PCT/JP2004/005891
(87) International publication number: WO 2004/094423

(57) **Abstract**

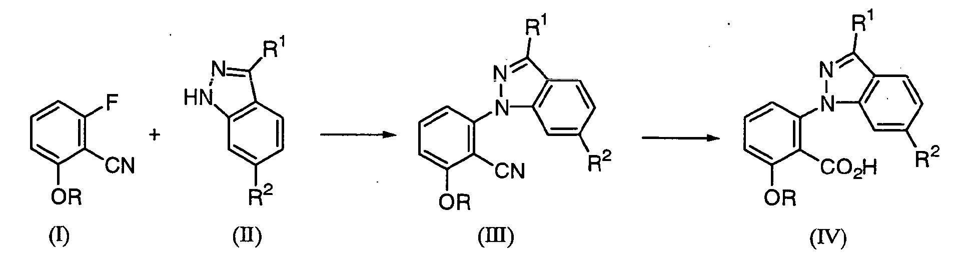

(wherein R represents lower alkyl; R¹ represents a hydrogen atom, -CH₂X or -OC(=O)R³; and R² represents a hydrogen atom, nitro, halogen, substituted or unsubstituted lower alkyl, substituted or unsubstituted lower alkoxy, substituted or unsubstituted lower alkylthio or substituted or unsubstituted aryl)

The present invention provides a simple industrial process for producing pyrazoloacridone derivatives having an antitumor activity, 1-(2-carboxyphenyl)indazole derivatives, which are useful as synthetic intermediates thereof, or the like; and the like.

For example, the present invention provides a process for producing a 1-(2-carboxyphenyl)indazole derivative represented by general formula (IV) which comprises steps of reacting a compound represented by above general formula (I) with a compound represented by above general formula (II) in the presence of a base to produce a compound represented by above general formula (III); and hydrolyzing a cyano group of the resulting compound represented by general formula (III).

## Description

### Technical Field

The present invention relates to a process for producing pyrazoloacridone derivatives having an antitumor activity, 1-(2-carboxyphenyl)indazole derivatives, which are useful as synthetic intermediates thereof, or the like; and the like.

### Background Art

It is known that pyrazoloacridone derivatives are useful as an antitumor agent [Journal of Medicinal Chemistry (J. Med. Chem.), Vol. 37, pp. 1028-1032 (1994) and Japanese Unexamined Patent Application Publication No. 1064/93]. Also, known processes for producing a pyrazoloacridone derivative include, for example, methods described in Japanese Unexamined Patent Application Publication No. 1064/93 and Japanese Unexamined Patent Application Publication No. 165758/95, and a method through the following synthetic intermediate [Japanese Unexamined Patent Application Publication No. 107641/94, Japanese Unexamined Patent Application Publication No. 76878/90, and Synthesis, pp. 73-76 (1994)].

It is known that a 1-(2-carboxyphenyl)indazole derivative represented by general formula (A): 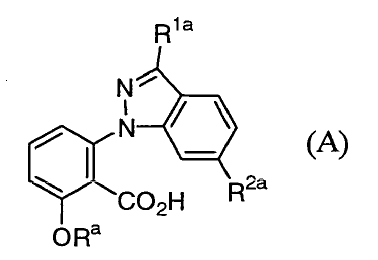
(wherein R^{a} represents methyl or the like; R^{1a} represents lower alkyl or the like; and R^{2a} represents nitro or the like), which is useful as synthetic intermediates of a pyrazoloacridone derivatives, is produced, for example, by reacting a benzoic acid derivative represented by general formula (B): 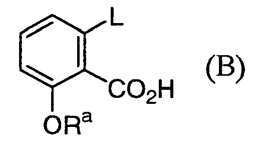
(wherein R^{a} has the same meaning as defined above; and L represents bromo or the like) with a compound represented by general formula (C): 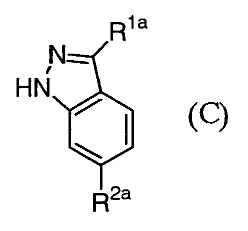
(wherein R^{1a} and R^{2a} have the same meanings as defined above, respectively) in the presence of a copper catalyst [Japanese Unexamined Patent Application Publication No. 107641/94 and Japanese Unexamined Patent Application Publication No. 76878/90 and Synthesis, pp. 73-76 (1994)]. However, the benzoic acid derivative represented by general formula (B) is difficult to obtain easily and in large quantities for industrial use, because it requires multiple synthetic steps, which include steps of protecting, deprotecting and the like, are required in the production, and it takes a long period of time for the production. Furthermore, since a copper catalyst is used in the above production process, an adverse effect on the environment by disposing waste liquid containing heavy metals and the like is of concern. Thus, the above production process includes problems as an industrial production process.

Accordingly, for industrial large-scale supply of desired 1-(2-carboxyphenyl)indazole derivatives, it is necessary to solve the above problems. In other words, the development of a process for producing a 1-(2-carboxyphenyl)indazole derivative through intermediates which can be efficiently produced by a small number of steps without using a copper catalyst has been desired.

### Disclosure of Invention

An object of the present invention is to provide a simple industrial process for producing pyrazoloacridone derivatives having an antitumor activity, 1-(2-carboxyphenyl)indazole derivatives, which are useful as synthetic intermediates thereof, or the like; synthetic intermediates thereof and the like.

The present invention relates to the following (1) to (14):
(1) A process for producing a pyrazoloacridone derivative represented by general formula (V): 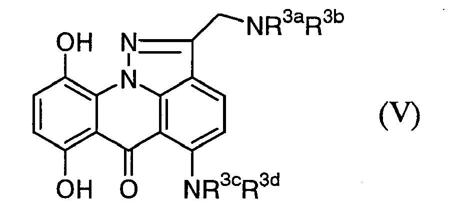
   <wherein R^{3a}, R^{3b}, R^{3c} and R^{3d} are the same or different and each represents a hydrogen atom, lower alkyl, -(CH₂)ₙ-Y¹ [wherein n represents an integer of 1 to 6; and Y¹ represents hydroxy, lower alkoxy, or -NR^{4a}R^{4b} {wherein R^{4a} and R^{4b} are the same or different and each represents a hydrogen atom, lower alkyl, or -(CH₂)ₘ-Y² [wherein m represents an integer of 1 to 6; and Y² represents hydroxy, lower alkoxy, or -NR^{5a}R^{5b} (wherein R^{5a} and R^{5b} are the same or different and each represents a hydrogen atom or lower alkyl)], or R^{4a} and R^{4b} forms a heterocyclic group together with the adjacent nitrogen atom}], or -CH((CH₂)ₚOH)₂ (wherein p represents an integer of 1 to 5)> which comprises steps of reacting a compound represented by general formula (I): 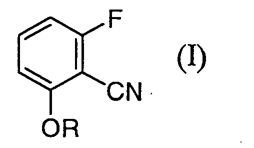
   (wherein R represents lower alkyl)
   with a compound represented by general formula (II): 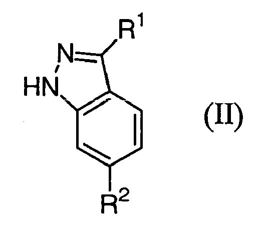
   [wherein R¹ represents a hydrogen atom, -CH₂X (wherein X represents a hydrogen atom, hydroxy, lower alkoxy or benzyloxy), or -OC(=O)R³ (wherein R³ represents lower alkyl); and R² represents a hydrogen atom, nitro, halogen, substituted or unsubstituted lower alkyl, substituted or unsubstituted lower alkoxy, substituted or unsubstituted lower alkylthio, or a substituted or unsubstituted aryl]
   in the presence of a base
   to produce a compound represented by general formula (III): 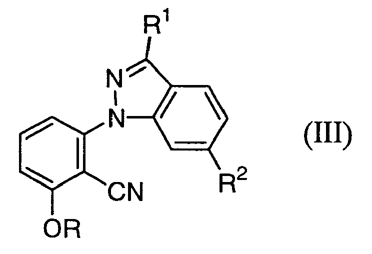
   (wherein R, R¹ and R² have the same meanings as defined above, respectively);
   and
   hydrolyzing a cyano group of the resulting compound represented by general formula (III)
   to produce a 1-(2-carboxyphenyl)indazole derivative represented by general formula (IV): 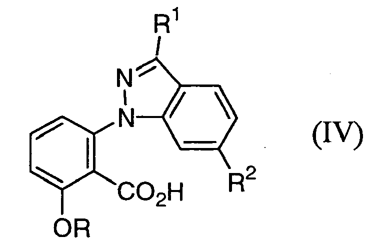
   (wherein R, R¹ and R² have the same meanings as defined above, respectively).
(2) A process for producing a pyrazoloacridone derivative represented by general formula (V): 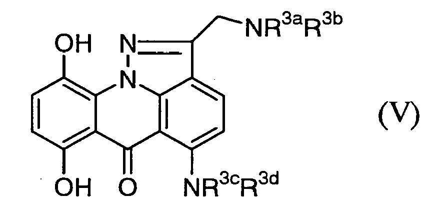
   (wherein R^{3a}, R^{3b}, R^{3c} and R^{3d} have the same meanings as defined above, respectively)
   which comprises steps of
   reacting 2,6-difluorobenzonitrile
   with a compound represented by general formula (II): 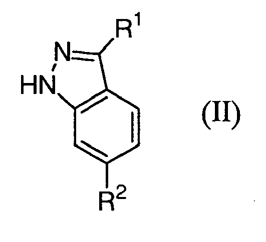
   (wherein R¹ and R² have the same meanings as defined above, respectively)
   in the presence of a base
   to produce a compound represented by general formula (VI): 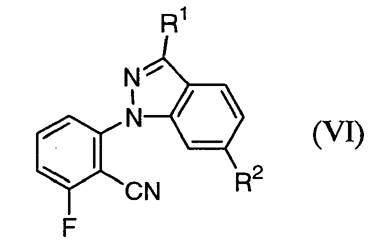
   (wherein R¹ and R² have the same meanings as defined above, respectively);
   converting the resulting compound represented by general formula (VI) into a compound represented by general formula (III) : 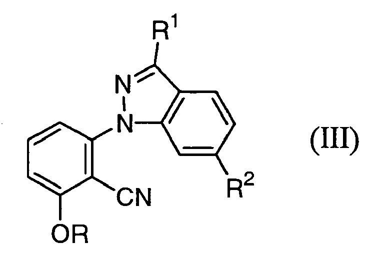
   (wherein R, R¹ and R² have the same meanings as defined above, respectively);
   and
   hydrolyzing a cyano group of the resulting compound represented by general formula (III)
   to produce a 1-(2-carboxyphenyl)indazole derivative represented by general formula (IV): 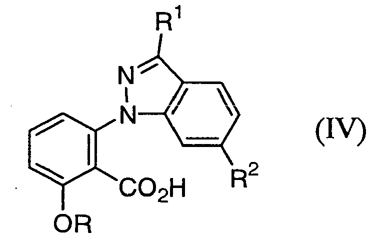
   (wherein R, R¹ and R² have the same meanings as defined above, respectively).
(3) The process for producing a pyrazoloacridone derivative according to the above (1) or (2), wherein R is methyl.
(4) The process for producing a pyrazoloacridone derivative according to any one of the above (1) to (3), wherein R¹ is lower alkyl; and R² is nitro or halogen.
(5) A process for producing a 1-(2-carboxyphenyl)indazole derivative represented by general formula (IV): 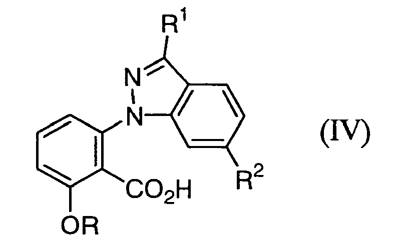
   (wherein R, R¹ and R² have the same meanings as defined above, respectively)
   which comprises steps of
   reacting a compound represented by general formula (I): 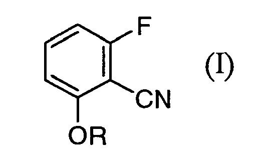
   (wherein R has the same meaning as defined above) with a compound represented by general formula (II): 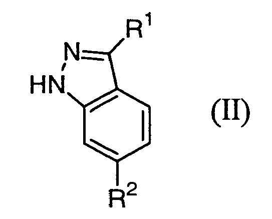
   (wherein R¹ and R² have the same meanings as defined above, respectively)
   in the presence of a base
   to produce a compound represented by general formula (III): 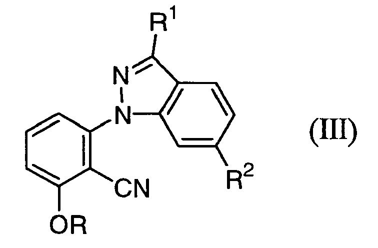
   (wherein R, R¹ and R² have the same meanings as defined above, respectively);
   and
   hydrolyzing a cyano group of the resulting compound represented by general formula (III).
(6) A process for producing a 1-(2-carboxyphenyl)indazole derivative represented by general formula (IV): 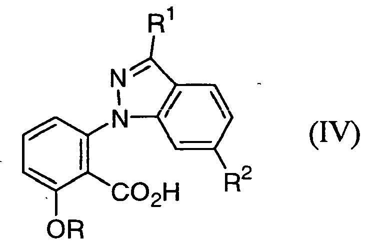
   (wherein R, R¹ and R² have the same meanings as defined above, respectively)
   which comprises
   reacting 2,6-difluorobenzonitrile
   with a compound represented by general formula (II): 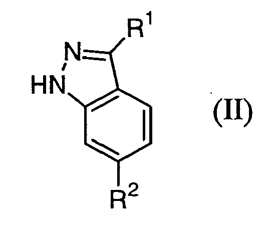
   (wherein R¹ and R² have the same meanings as defined above, respectively)
   in the presence of a base
   to produce a compound represented by general formula (VI): 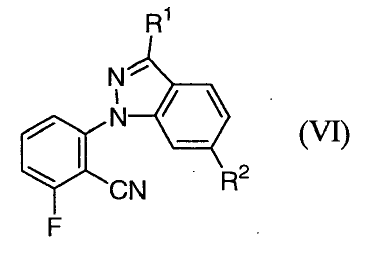
   (wherein R¹ and R² have the same meanings as defined above, respectively);
   converting the resulting compound represented by general formula (VI) into a compound represented by general formula (III) : 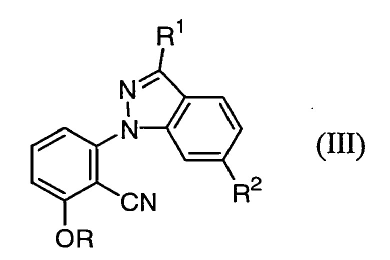
   (wherein R, R¹ and R² have the same meanings as defined above, respectively);
   and
   hydrolyzing a cyano group of the resulting compound represented by general formula (III).
(7) The process for producing a 1-(2-carboxyphenyl)indazole derivative according to the above (5) or (6), wherein R is methyl.
(8) The process for producing a 1-(2-carboxyphenyl)indazole derivative according to any one of the above (5) to (7), wherein R¹ is lower alkyl; and R² is nitro or halogen.
(9) A compound represented by general formula (III): 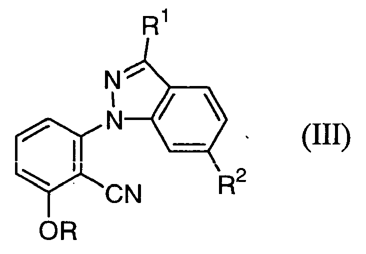
   (wherein R, R¹ and R² have the same meanings as defined above, respectively)
   or a salt thereof.
(10) The compound according to the above (9), wherein R is methyl, or a salt thereof.
(11) A process for producing a compound represented by general formula (III): 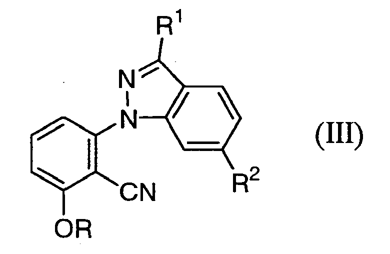
   (wherein R, R¹ and R² have the same meanings as defined above, respectively)
   which comprises reacting a compound represented by general formula (I): 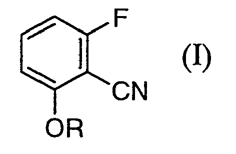
   (wherein R has the same meaning as defined above)
   with a compound represented by general formula (II): 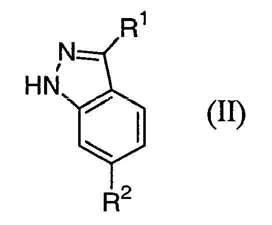
   (wherein R¹ and R² have the same meanings as defined above, respectively)
   in the presence of a base.
(12) The process according to the above (11), wherein R is methyl.
(13) A compound represented by general formula (VI): 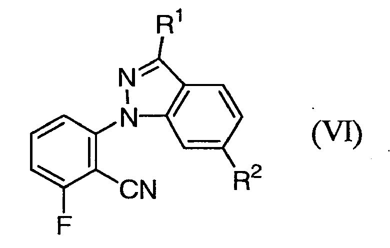
   (wherein R¹ and R² have the same meanings as defined above, respectively)
   or a salt thereof.
(14) A process for producing a compound represented by general formula (VI): 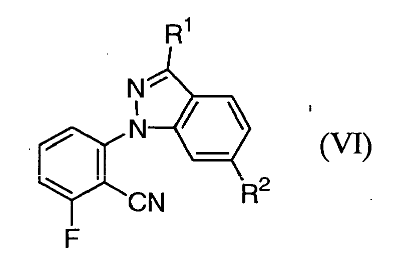
   (wherein R¹ and R² have the same meanings as defined above, respectively)
   which comprises
   reacting 2,6-difluorobenzonitrile
   with a compound represented by general formula (II): 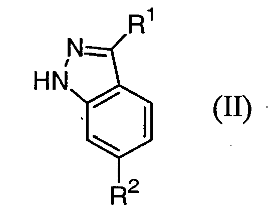
   (wherein R¹ and R² have the same meanings as defined above, respectively)
   in the presence of a base.

Hereinafter, the compound represented by general formula (I) is referred to as Compound (I). The same applies to the compounds of the other formula numbers.

In the definition of each group in general formulae (I), (II), (III), (IV), (V) and (VI):

Examples of the lower alkyl moiety in the lower alkyl, the lower alkoxy and the lower alkylthio include straight-chain or branched alkyl groups having 1 to 6 carbon atoms, such as methyl, ethyl, propyl, isopropyl, butyl, sec-butyl, tert-butyl, pentyl, isopentyl, neopentyl and hexyl.

Examples of the aryl include aryls having 6 to 14 carbon atoms, such as phenyl, naphthyl and anthryl.

The halogen means an atom of fluorine, chlorine, bromine or iodine.

Examples of the heterocyclic group formed together with the adjacent nitrogen atom include pyrrolidinyl, piperidino, piperazinyl, morpholino, thiomorpholino, quinolyl, pyrimidinyl, pyridazinyl, pyridyl, pyrrolyl, imidazolyl and pyrazolyl.

Examples of the substituents in the substituted lower alkyl, the substituted lower alkoxy or the substituted lower alkylthio include halogen. Herein, the halogen has the same meaning as the above-described halogen.

Examples of the substituents in the substituted aryl are the same or different and include 1 to 3 substituent(s), such as lower alkyl, lower alkoxy and halogen. Herein, the lower alkyl, the lower alkoxy and the halogen have the same meanings as the above-described lower alkyl, lower alkoxy and halogen, respectively.

Examples of processes for producing Compound (III), Compound (IV), Compound (V) and Compound (VI) in the present invention will be described below.

### Production process 1

Compound (IV) can be produced from Compound (I) according to the following series of reaction steps: 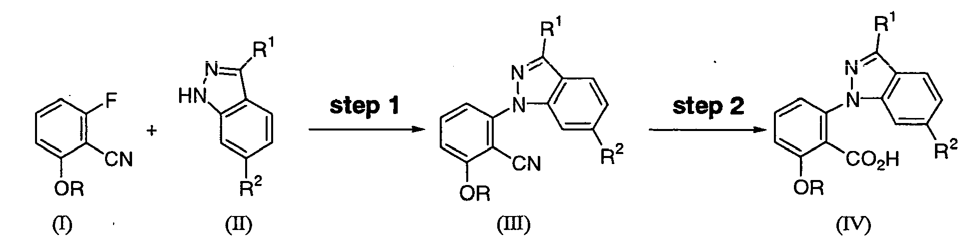
(wherein R, R¹ and R² have the same meanings as defined above, respectively)

### Step 1:

Compound (III) can be obtained by reacting Compound (I) with Compound (II) in an inert solvent in the presence of 1 to 5 equivalents, preferably 1 to 2 equivalents of a base based on Compound (II).

Compound (I), which is a raw material, can be commercially available, or can be produced from 2,6-difluorobenzonitrile, which can be obtained easily and in large quantities for industrial use, by a method described in, for example, Journal of Heterocyclic Chemistry (J. Heterocyclic Chem.), Vol. 25, pp. 1173-1177 (1988), or methods similar to that.

Compound (II), which is a raw material, can be produced by a method described in, for example, Journal of American Chemical Society (J. Am. Chem. Soc.), Vol. 74, pp. 2009-2012 (1952) or Chemical Abstracts (CA), Vol. 65, 2245b (1966), or methods similar to those.

Examples of the base include hydroxides of an alkali metal atom such as sodium hydroxide, potassium hydroxide, and lithium hydroxide; hydroxides of an alkaline earth metal atom such as calcium hydroxide and magnesium hydroxide; carbonates of an alkali metal atom such as sodium carbonate, sodium hydrogencarbonate, potassium carbonate and potassium hydrogencarbonate; carbonates of an alkaline earth metal atom such as calcium carbonate; hydrides of an alkali metal such as sodium hydride, potassium hydride and lithium hydride; and hydrides of an alkaline earth metal such as calcium hydride. These may be used alone or in combination. Among them, sodium carbonate, potassium carbonate or sodium hydride is preferable.

Examples of the inert solvent include amide solvents such as N,N-dimethylformamide (DMF), N,N-dimethylacetamide (DMA) and N-methylpyrrolidone (NMP); and polar solvents such as N,N-dimethylimidazolidinone (DMI) and dimethylsulfoxide (DMSO). These may be used alone or in combination. Among them, DMF or NMP is preferable.

The reaction is generally carried out at a temperature between 50°C and 150°C, preferably 80°C and 120°C, for 1 to 12 hours, preferably 1 to 8 hours.

Compound (I) is used in an amount of 0.8 to 2.0 equivalents, preferably 1.0 to 1.5 equivalents based on Compound (II).

### Step 2:

Compound (IV) can be obtained by treating Compound (III) obtained in step 1 in a solvent in the presence of an acid catalyst.

Examples of the solvent include water and mixed solvents containing water and an alcohol solvent such as methanol and ethanol. When the mixed solvent is used, the mixing ratio of the alcohol solvent to water is preferably 50 weight/weight % or less, particularly preferably 20 weight/weight % or less.

Examples of the acid catalyst include inorganic acids such as hydrochloric acid and sulfuric acid. Among them, sulfuric acid is preferable. The concentration of the acid catalyst in the solvent is preferably 10 to 90 weight/weight %, particularly preferably 40 to 70 weight/weight %.

The reaction is generally carried out at a temperature between 30°C and 100°C, preferably 70°C and 100°C, for 5 minutes to 24 hours, preferably 10 minutes to 6 hours.

### Production process 2

Compound (III), which is an intermediate in Production process 1, can also be produced from 2,6-difluorobenzonitrile according to the following series of reaction steps: 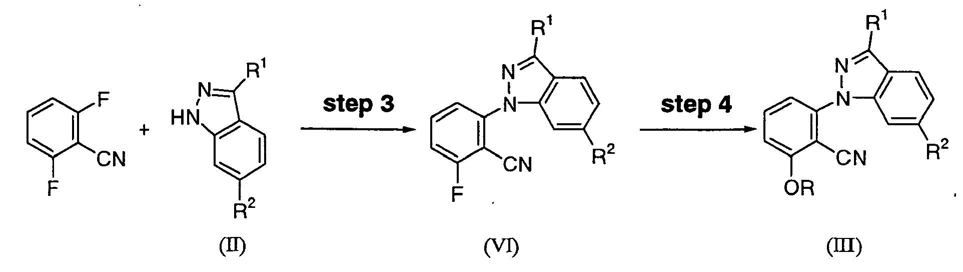
(wherein R, R¹ and R² have the same meanings as defined above, respectively)

### Step 3:

Compound (VI) can be obtained by reacting 2,6-difluorobenzonitrile with Compound (II) in an inert solvent in the presence of 1 to 5 equivalents, preferably 1 to 2 equivalents of a base based on Compound (II).

2,6-Difluorobenzonitrile, which is a raw material, can be commercially available and can be obtained easily and in large quantities for industrial use.

Examples of the base include hydroxides of an alkali metal atom such as sodium hydroxide, potassium hydroxide and lithium hydroxide; hydroxides of an alkaline earth metal atom such as calcium hydroxide and magnesium hydroxide; carbonates of an alkali metal atom such as sodium carbonate, sodium hydrogencarbonate, potassium carbonate and potassium hydrogencarbonate; carbonates of an alkaline earth metal atom such as calcium carbonate; hydrides of an alkali metal such as sodium hydride, potassium hydride and lithium hydride; and hydrides of an alkaline earth metal such as calcium hydride. These may be used alone or in combination. Among them, sodium carbonate, potassium carbonate or sodium hydride is preferable.

Examples of the inert solvent include amide solvents such as DMF, DMA, and NMP; and polar solvents such as DMI and DMSO. These may be used alone or in combination. Among them, DMF is preferable.

The reaction is generally carried out at a temperature between 50°C and 150°C, preferably 80°C and 120°C, for 1 to 10 hours, preferably 1 to 8 hours.

2,6-difluorobenzonitrile is used in an amount of 1.0 to 20.0 equivalents, preferably 1.0 to 5.0 equivalents based on Compound (II).

### Step 4:

Compound (III) can be obtained by treating Compound (VI) obtained in step 3 in a solvent with 1 to 50 equivalents, preferably 1 to 20 equivalents of an alkoxylation agent.

Examples of the alkoxylation agent include MOR (wherein M represents lithium, potassium or sodium; and R has the same meaning as defined above). Specifically, when R is methyl, a solid of lithium methoxide (LiOCH₃), sodium methoxide (NaOCH₃) or potassium methoxide (KOCH₃); a methanol solution thereof; or the like can be used.

Examples of the solvent include amide solvents such as DMF, DMA, and NMP; ether solvents such as tetrahydrofuran (THF); and polar solvents such as DMI, DMSO and methanol. These may be used alone or in combination. Among them, DMF, DMA, THF or methanol, or mixed solvents thereof is preferable.

The reaction is generally carried out at a temperature between 50°C and the boiling point of a solvent used in the reaction, preferably 70°C and 120°C, or at the boiling point of the solvent used in the reaction, for 1 to 16 hours, preferably 1 to 10 hours.

### Production process 3

Compound (V) can be produced from Compound (IV) obatined in Production process 1 by a method described in, for example, Japanese Unexamined Patent Application Publication No. 76878/90, Synthesis, pp. 73-76 (1994), Japanese Unexamined Patent Application Publication No. 1064/93 or Japanese Unexamined Patent Application Publication No.165758/95, or methods similar to those.

The intermediates and the desired compounds produced in the above-described production processes can be isolated and purified by separation and purification methods which are generally used in synthetic organic chemistry, such as filtration, extraction, washing, drying, concentration, recrystallization and various chromatographies. The intermediates can be subjected to the subsequent reaction without a particular purification.

Typical examples of the present invention will now be described below, but the present invention is not limited to these examples.

### Best Mode for Carrying Out the Invention

### EXAMPLE 1: Synthesis of 1-(2-cyano-3-methoxyphenyl)-3-methyl-6-nitroindazole (Compound 1)

2-Fluoro-6-methoxybenzonitrile (9.38 g), 3-methyl-6-nitroindazole (10.0 g) and powdered potassium carbonate (7.80 g) were stirred in DMF (100 mL) in an atmosphere of nitrogen at 100°C for 6 hours. Water (100 mL) was added dropwise to the reaction mixture while the reaction system was kept at 80°C. DMF (50 mL) was added to the suspension and the mixture was cooled to room temperature over a period of 4 hours while stirring. The precipitated crystals were recovered by filtration and the resulting crystals were washed with water (100 mL). Subsequently, the crystals were dried under reduced pressure to give Compound 1 (15.1 g, yield 86.7%) as pale yellow crystals.
Melting point: 244.0°C
¹H-NMR (300 MHz, CDCl₃) δ (ppm): 8.36 (1H, d, J = 1.8 Hz), 8.11 (1H, dd, J = 1.8, 8.8 Hz), 7.86 (1H, d, J = 8.8 Hz), 7.73 (1H, t, J = 8.6 Hz), 7.23 (1H, d, J = 8.6 Hz), 7.11 (1H, d, J = 8.6 Hz), 4.06 (3H, s), and 2.72 (3H, s).
¹³C-NMR (75 MHz, CDCl₃) δ (ppm): 163.1, 147.5, 145.8, 142.1, 139.3, 134.8, 128.1, 121.6, 118.1, 116.4, 113.4, 110.8, 106.9, 99.5, 56.7, and 12.0.
IR (KBr, cm⁻¹): 2,849 (OCH₃), 2,228 (CN), 1,528 and 1,346 (NO₂). HRMS (ESI⁺): calculated value (C₁₆H₁₃N₄O₃); 309.0988, measured value; 309.0979.

### EXAMPLE 2: Synthesis of 1-(2-carboxy-3-methoxyphenyl)-3-methyl-6-nitroindazole (Compound 2)

Compound 1 (1.00 g) obtained in Example 1 was added to an aqueous solution of 50% sulfuric acid (40 mL), and the mixture was stirred at 80°C for 1 hour. The reaction mixture was poured into water (200 mL) little by little while stirring. The white suspension was stirred at 0°C for more than 2 hours. The precipitated crystals were recovered by filtration and the resulting crystals were washed with water. Subsequently, the crystals were dried at 40°C under reduced pressure to give Compound 2 (0.972 g, yield 91.6%).
¹H-NMR (300 MHz, DMSO-d₆) δ (ppm): 8.22 (1H, dd, J = 0.6, 1.8 Hz), 8.10 (1H, dd, J = 0.6, 8.8 Hz), 8.04 (1H, dd, J = 1.8, 8.8 Hz), 7.65 (1H, t, J = 7.9 Hz), 7.31 (1H, d, J = 7.9 Hz), 7.29 (1H, d, J = 7.9 Hz), 3.90 (3H, s), and 2.62 (3H, s).

### EXAMPLE 3: Synthesis of 1-(2-cyano-3-fluorophenyl)-3-methyl-6-nitroindazole (Compound 3)

2,6-Difluorobenzonitrile (5.89 g), 3-methyl-6-nitroindazole (5.00 g) and powdered potassium carbonate (11.7 g) were stirred in DMF (85 mL) in an atmosphere of nitrogen at 90°C for 5 hours. The reaction mixture was cooled to room temperature, and a precipitated insoluble substance was removed by filtration. The DMF was distilled away from the resulting filtrate under reduced pressure. The resulting residue was dissolved in ethyl acetate, water was added to the solution, and then the mixture was separated. The organic layer was sequentially washed with water and a saturated brine solution and was dried over anhydrous magnesium sulfate. The ethyl acetate was distilled away under reduced pressure. Subsequently, the resulting residue was purified by silica gel column chromatography (eluate: ethyl acetate/hexane = 40/60) to give Compound 3 (2.72 g, yield 32.5%) as crystals.
Melting point: 199.7°C
¹H-NMR (300 MHz, CDCl₃) δ (ppm): 8.41 (1H, d, J = 2.0 Hz), 8.16 (1H, dd, J = 2.0, 8.8 Hz), 7.89 (1H, d, J = 8.8 Hz), 7.81 (1H, dt, J = 6.0, 8.3 Hz), 7.51 (1H, d, J = 8.3 Hz), 7.35 (1H, dt, J = 0.9, 8.3 Hz), and 2.73 (3H, s).
¹³C-NMR (75 MHz, CDCl₃) δ(ppm): 164.5 (d, ¹J = 262 Hz), 147.7, 146.5, 141.8 (d, ³J = 2.5 Hz), 139.1, 135.3 (d, ³J = 10.0 Hz), 128.4, 121.9, 121.1 (d, ⁴J = 3.8 Hz), 116.9, 115.6 (d, ²J = 19.9 Hz), 111.2, 106.6, 99.3 (d, ²J = 17.4 Hz), and 12.0.
IR (KBr, cm⁻¹): 2,233 (CN), 1,535 and 1,350 (NO₂).

### EXAMPLE 4: Synthesis of 1-(2-cyano-3-methoxyphenyl)-3-methyl-6-nitroindazole (Compound 1)

Compound 3 (100 mg) synthesized in Example 3 was suspended in a mixed solvent including methanol (5.0 mL) and THF (5.0 mL). A 28% methanol solution of sodium methoxide (1.30 g) was added to the suspension, and then the mixture was refluxed under heating at 75°C to 80°C for 7 hours. The solvent was distilled away under reduced pressure. Subsequently, methanol (5.0 mL) and water (5.0 mL) were added to the residue. The precipitated crystals were recovered by filtration and the resulting crystals were washed with water (10 mL). Subsequently, the crystals were dried under reduced pressure to give Compound 1 (96.6 mg, yield 92.7%) as pale yellow crystals. It was confirmed that Compound 1 synthesized by this reaction was the same as the compound obtained in Example 1 by a measurement of ¹H-NMR spectrum.

### Industrial Applicability

According to the present invention, a simple industrial process for producing pyrazoloacridone derivatives having an antitumor activity, 1-(2-carboxyphenyl)indazole derivatives, which are useful as synthetic intermediates thereof, or the like; synthetic intermediates thereof and the like are provided.

## Claims

1. A process for producing a pyrazoloacridone derivative represented by general formula (V): 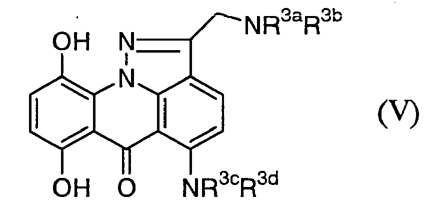
<wherein R^{3a}, R^{3b}, R^{3c} and R^{3d} are the same or different and each represents a hydrogen atom, lower alkyl, -(CH₂)ₙ-Y¹ [wherein n represents an integer of 1 to 6; and Y¹ represents hydroxy, lower alkoxy, or -NR^{4a}R^{4b} {wherein R^{4a} and R^{4b} are the same or different and each represents a hydrogen atom, lower alkyl, or -(CH₂)ₘ-Y² [wherein m represents an integer of 1 to 6; and Y² represents hydroxy, lower alkoxy, or -NR^{5a}R^{5b} (wherein R^{5a} and R^{5b} are the same or different and each represents a hydrogen atom or lower alkyl)], or R^{4a} and R^{4b} forms a heterocyclic group together with the adjacent nitrogen atom}], or -CH((CH₂)ₚOH)₂ (wherein p represents an integer of 1 to 5)> which comprises steps of
reacting a compound represented by general formula (I): 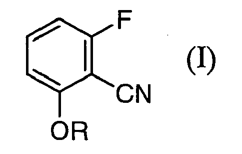
(wherein R represents lower alkyl)
with a compound represented by general formula (II): 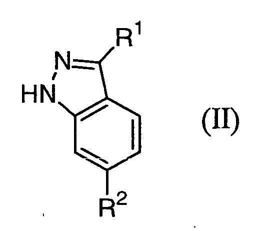
[wherein R¹ represents a hydrogen atom, -CH₂X (wherein X represents a hydrogen atom, hydroxy, lower alkoxy or benzyloxy), or -OC(=O)R³ (wherein R³ represents lower alkyl); and R² represents a hydrogen atom, nitro, halogen, substituted or unsubstituted lower alkyl, substituted or unsubstituted lower alkoxy, substituted or unsubstituted lower alkylthio, or a substituted or unsubstituted aryl]
in the presence of a base
to produce a compound represented by general formula (III): 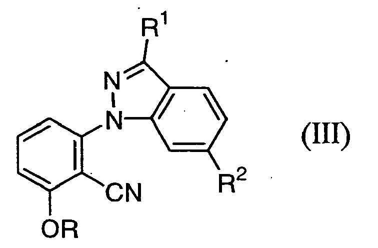
(wherein R, R¹ and R² have the same meanings as defined above, respectively);
and
hydrolyzing a cyano group of the resulting compound represented by general formula (III)
to produce a 1-(2-carboxyphenyl)indazole derivative represented by general formula (IV): 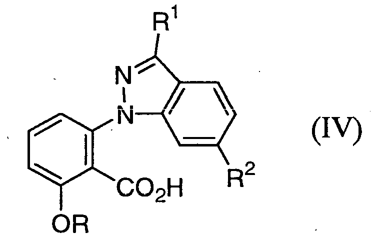
(wherein R, R¹ and R² have the same meanings as defined above, respectively).

2. A process for producing a pyrazoloacridone derivative represented by general formula (V): 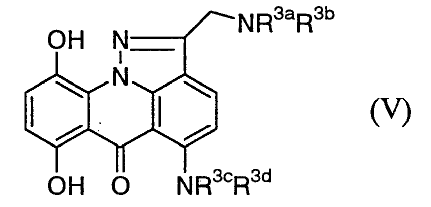
(wherein R^{3a}, R^{3b}, R^{3c} and R^{3d} have the same meanings as defined above, respectively)
which comprises steps of
reacting 2,6-difluorobenzonitrile
with a compound represented by general formula (II): 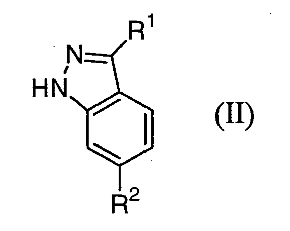
(wherein R¹ and R² have the same meanings as defined above, respectively)
in the presence of a base
to produce a compound represented by general formula (VI): 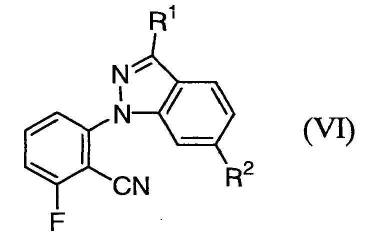
(wherein R¹ and R² have the same meanings as defined above, respectively);
converting the resulting compound represented by general formula (VI) into a compound represented by general formula (III) : 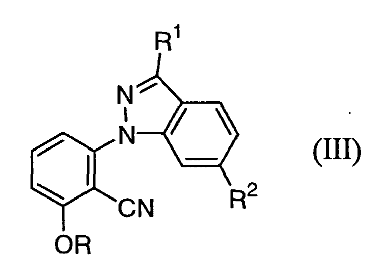
(wherein R, R¹ and R² have the same meanings as defined above, respectively);
and
hydrolyzing a cyano group of the resulting compound represented by general formula (III)
to produce a 1-(2-carboxyphenyl)indazole derivative represented by general formula (IV): 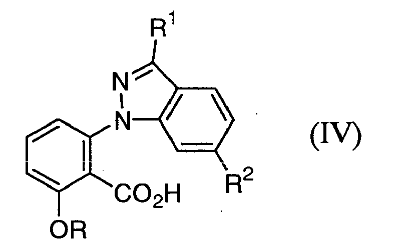
(wherein R, R¹ and R² have the same meanings as defined above, respectively).

3. The process for producing a pyrazoloacridone derivative according to claim 1 or 2, wherein R is methyl.

4. The process for producing a pyrazoloacridone derivative according to any one of claims 1 to 3, wherein R¹ is lower alkyl; and R² is nitro or halogen.

5. A process for producing a 1-(2-carboxyphenyl)indazole derivative represented by general formula (IV): 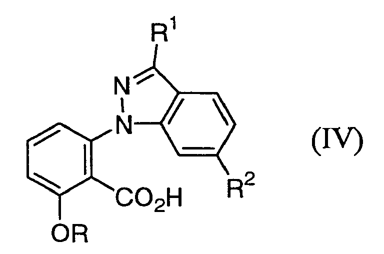
(wherein R, R¹ and R² have the same meanings as defined above, respectively)
which comprises steps of
reacting a compound represented by general formula (I): 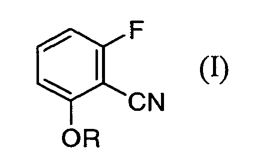
(wherein R has the same meaning as defined above)
with a compound represented by general formula (II): 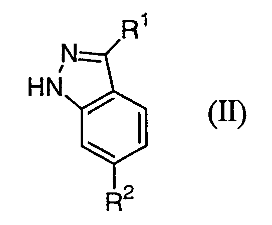
(wherein R¹ and R² have the same meanings as defined above, respectively)
in the presence of a base
to produce a compound represented by general formula (III): 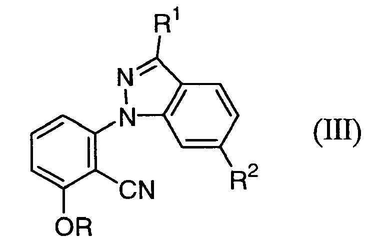
(wherein R, R¹ and R² have the same meanings as defined above, respectively);
and
hydrolyzing a cyano group of the resulting compound represented by general formula (III).

6. A process for producing a 1-(2-carboxyphenyl)indazole derivative represented by general formula (IV): 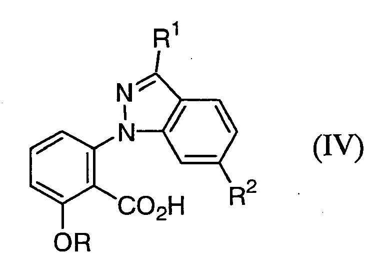
(wherein R, R¹ and R² have the same meanings as defined above, respectively)
which comprises
reacting 2,6-difluorobenzonitrile
with a compound represented by general formula (II): 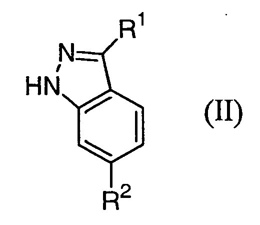
(wherein R¹ and R² have the same meanings as defined above, respectively)
in the presence of a base
to produce a compound represented by general formula (VI): 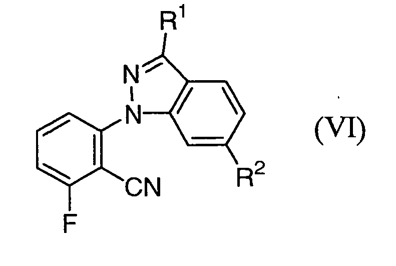
(wherein R¹ and R² have the same meanings as defined above, respectively);
converting the resulting compound represented by general formula (VI) into a compound represented by general formula (III) : 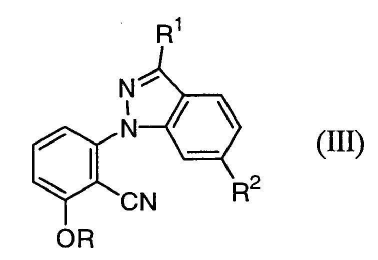
(wherein R, R¹ and R² have the same meanings as defined above, respectively);
and
hydrolyzing a cyano group of the resulting compound represented by general formula (III).

7. The process for producing a 1-(2-carboxyphenyl)indazole derivative according to claim 5 or 6, wherein R is methyl.

8. The process for producing a 1-(2-carboxyphenyl)indazole derivative according to any one of claims 5 to 7, wherein R¹ is lower alkyl; and R² is nitro or halogen.

9. A compound represented by general formula (III): 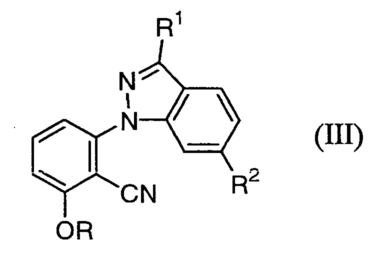
(wherein R, R¹ and R² have the same meanings as defined above, respectively)
or a salt thereof.

10. The compound according to claim 9, wherein R is methyl, or a salt thereof.

11. A process for producing a compound represented by general formula (III): 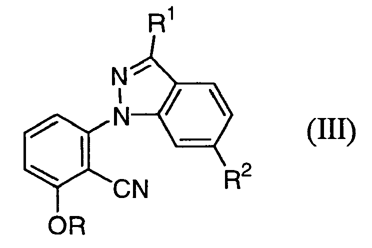
(wherein R, R¹ and R² have the same meanings as defined above, respectively)
which comprises
reacting a compound represented by general formula (I): 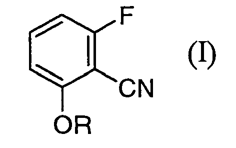
(wherein R has the same meaning as defined above)
with a compound represented by general formula (II): 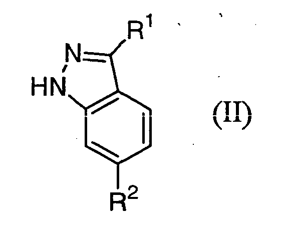
(wherein R¹ and R² have the same meanings as defined above, respectively)
in the presence of a base.

12. The process according to claim 11, wherein R is methyl.

13. A compound represented by general formula (VI): 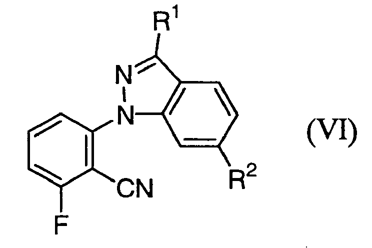
(wherein R¹ and R² have the same meanings as defined above, respectively)
or a salt thereof.

14. A process for producing a compound represented by general formula (VI): 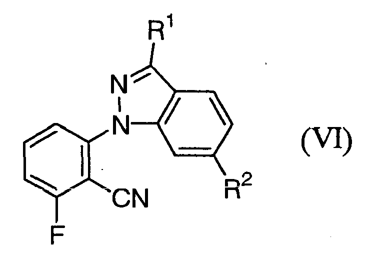
(wherein R¹ and R² have the same meanings as defined above, respectively)
which comprises
reacting 2,6-difluorobenzonitrile
with a compound represented by general formula (II): 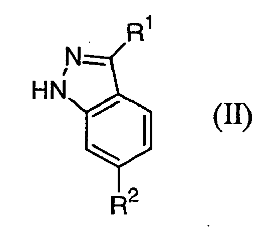
(wherein R¹ and R² have the same meanings as defined above, respectively)
in the presence of a base.
